# FASCICULE DE BREVET EUROPEEN

(11) **EP 0 793 957 B1**
(45) Date de publication et mention de la délivrance du brevet: **30.12.1998**
(21) Numéro de dépôt: 97400177.8
(22) Date de dépôt: 27.01.1997
(51) Int. Cl.: A61K 7/48

(54) **Composition cosmétique comprenant une dispersion aqueuse de particules polymériques et utilisation de la dite composition**
Kosmetische Zusammensetzung enthaltend eine wässrige Dispersion von Polymerteilchen und Verwendung
Cosmetic composition comprising an aqueous dispersion of polymeric particles and use thereof

(30) Priorité: 04.03.1996 FR 9602682
(43) Date de publication de la demande: 10.09.1997
(73) Titulaire: L'OREAL, 75008 Paris (FR)
(72) Inventeur: de la Poterie, Valérie, 77820 le Chatelet en Brie (FR); Bara, Isabelle, 75013 Paris (FR)
(74) Mandataire: Dodin, Catherine

(56) Documents cités:
- EP-A- 0 265 228
- EP-A- 0 655 234
- WO-A-92/19215
- FR-A- 2 229 393
- FR-A- 2 679 769
- US-A- 3 639 572
- US-A- 4 423 031
- US-A- 4 795 631
- PATENT ABSTRACTS OF JAPAN vol. 016, no. 552 (C-1006), 20 Novembre 1992 & JP 04 210613 A (KAO CORP), 31 Juillet 1992,

## Description

La présente invention a trait à une composition notamment cosmétique susceptible d'être appliquée sur la peau, les semi-muqueuses et/ou les muqueuses. Ladite composition comprend en particulier une dispersion aqueuse de particules de polymère filmogène et peut être utilisée en tant que produit de maquillage.

Les compositions à appliquer sur la peau et/ou les muqueuses, de type rouge à lèvres ou fond de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.
Ces compositions, lorsqu'elles sont appliquées sur la peau, présentent toutefois l'inconvénient de transférer. On entend par là le fait que la composition est susceptible de se déposer, au moins en partie, sur certains supports avec lesquels elle est mise en contact, tels que, par exemple, un verre, un vêtement ou la peau. En se déposant, ladite composition laisse une trace sur ledit support.
Il s'en suit donc une persistance médiocre de la composition sur la peau ou les muqueuses, et la nécessité de renouveler régulièrement son application.
Un autre inconvénient des compositions de l'art antérieur réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. On a encore constaté que les eye-liners pouvaient également couler. Tous ces phénomènes engendrent un effet inesthétique, que la consommatrice souhaite bien évidemment éviter.
On connaît des compositions de maquillage dites 'sans transfert', susceptibles de pallier les inconvénients ci-dessus mentionnés; elles comprennent généralement, parmi les corps gras qui les composent, des huiles volatiles, notamment des huiles de silicone volatiles et/ou des huiles hydrocarbonées volatiles. Toutefois, ces compositions conduisent à un maquillage très mat.

La présente invention a pour but de proposer une composition qui permet d'obtenir un film de très bonne tenue, qui ne transfère pas et ne tache pas un support avec lequel il serait en contact, et qui ne migre pas au cours du temps, tout en permettant d'obtenir un maquillage et/ou un film brillant.

Ainsi, un objet de l'invention est l'utilisation dans une composition susceptible d'être appliquée sur la peau, les semi-muqueuses et/ou les muqueuses, d'un système polymérique comprenant une dispersion aqueuse de particules de polymère filmogène, pour l'obtention sur un support d'un film ayant une dureté inférieure à environ 110.

Par ailleurs, il est connu du document US-A-4795631, une composition pour les lèvres comprenant une résine filmogène, solubilisée ou dispersée dans un solvant organique volatil ; du document FR-A-2229393, une composition filmogène pour la peau comprenant une émulsion de polymère filmogène insoluble dans l'eau ; du document US-4423031, un eyeliner comprenant un polymère filmogène en émulsion et du document US-A-4639572, une composition pour la peau contenant une dispersion de copolymère filmogène insoluble dans l'eau.

Un autre objet est l'utilisation pour maquiller, protéger et/ou traiter non thérapeutiquement la peau, les semi-muqueuses et/ou les muqueuses, en particulier les lèvres du visage, d'un système polymérique ou d'une composition le comprenant, ledit système comprenant une dispersion aqueuse de particules de polymère filmogène et étant tel qu'il permette l'obtention sur un support d'un film ayant une dureté inférieure à environ 110.
Un autre objet est une composition susceptible d'être appliquée sur la peau, les semi-muqueuses et/ou les muqueuses, et notamment une composition de rouge à lèvres, comprenant un système polymérique qui comprend une dispersion aqueuse de particules de polymère filmogène, ledit système polymérique étant tel qu'il permette l'obtention sur un support d'un film ayant une dureté inférieure à environ 110.

On a constaté que la composition selon l'invention est facilement applicable et s'étale aisément et uniformément sur la peau, les semi-muqueuses et les muqueuses, en particulier sur les lèvres du visage.
Elle permet l'obtention d'un film homogène, qui présente une texture légère et reste confortable à porter tout au long de la journée. Le film n'est pas du tout collant, tout en étant mou, souple, élastique et flexible sur la peau; il suit les mouvements du support sur lequel il est déposé, sans se craqueler et/ou se décoller. Il adhère notamment parfaitement sur les lèvres du visage. La composition selon l'invention trouve donc une application particulièrement intéressante en tant que composition à appliquer sur les lèvres, et notamment en tant que rouge à lèvres. La composition selon l'invention trouve également une autre application avantageuse dans le domaine des eye-liners.
D'autre part, le film obtenu peut être très brillant, ou plus ou moins mat, selon la nature des constituants de la composition, d'où une gamme plus étendue de produits de maquillage, brillants ou mats, au choix.

La composition selon l'invention comprend donc un système polymérique qui comprend au moins une dispersion aqueuse de particules de polymère filmogène. Parmi les polymères filmogènes utilisables dans le cadre de la présente invention, on peut citer les polymères synthétiques, de type polycondensat ou de type radicalaires, les polymères d'origine naturelle, et leurs mélanges.

On peut ainsi citer, parmi les polycondensats, les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, et leurs mélanges.
Le polyuréthanne peut être, par exemple, un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.
Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.
On peut également citer les polyesters, les polyesters amides, les polyesters à chaîne grasse, les polyamides, et les résines époxyesters.
Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylène diamine, l'hexaméthylènediamine, la méta- ou para-phénylène diamine. Comme aminoalcool, on peut utiliser la monoéthanolamine.
Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.
Les polyesters à chaîne grasse peuvent être obtenus par l'utilisation de diols à chaîne grasse lors de la polycondensation.
Les résines époxyesters peuvent être obtenues par polycondensation d'acides gras avec un condensat aux extrémités α, ω - diépoxy.

Les polymères de type radicalaires peuvent être notamment des polymères, ou des copolymères, acryliques et/ou vinyliques. On utilise de préférence des polymères radicalaires anioniques.

Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.
Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxy propyle, le méthacrylate d'hydroxyéthyle, le méthacrylate de 2-hydroxy propyle.
Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.
On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.
On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur base de ses connaissances générales.

Afin d'améliorer le caractère filmogène d'un polymère, par exemple en abaissant sa température de transition vitreuse, il est possible d'ajouter à la dispersion un agent de coalescence, qui peut être choisi parmi les agents de coalescence connus.
Dans la présente description, on entend par'dispersion de polymère filmogène', une dispersion susceptible de former un film, comprenant ou ne comprenant pas d'agent de coalescence.

La teneur en matière sèche desdites dispersions aqueuses selon la présente invention peut être de l'ordre de 5-60% en poids, et de préférence 30-40%.

La composition peut comprendre 1-60% en poids, de préférence 5-40% en poids de matière sèche de polymères filmogènes.

La taille des particules de polymères en dispersion aqueuse peut être comprise entre 10-500 nm, et est de préférence comprise entre 20 et 150 nm, ce qui permet d'obtenir un film ayant une brillance remarquable.

Afin de réaliser la présente invention, il est donc nécessaire que le système polymérique permette l'obtention d'un film sur le support sur lequel il est déposé, ledit film devant avoir une dureté inférieure à environ 110. De préférence, le film a une dureté inférieure à 70, et préférentiellement inférieure à 55.
D'autre part, dans un mode de réalisation préféré, ledit système polymérique est choisi de manière à permettre l'obtention d'un film ayant une élongation supérieure à environ 200%, et, de manière préférentielle, supérieure à 300%.
Les méthodes de mesure d'élongation et de dureté sont décrites avant les exemples.

Afin d'obtenir la dureté, et éventuellement l'élongation, souhaitées, on peut utiliser un composés susceptible d'influencer sur ces caractéristiques, à savoir un agent plastifiant.
Le système polymérique selon l'invention comprend alors la dispersion aqueuse de particules de polymère filmogène et l'agent plastifiant.
Ledit agent plastifiant peut être choisi parmi tous les composés connus de l'homme du métier comme étant susceptibles de remplir la fonction recherchée. Cet agent peut être hydrosoluble ou insoluble dans l'eau et peut éventuellement se présenter sous forme de dispersion aqueuse.
En particulier, on peut citer, seuls ou en mélange, les plastifiants usuels, tels que:
. les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
. les esters de glycérol,
. les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
. des esters d'acides tels que des citrates, des phtalates, des adipates, des carbonates, des tartrates, des phosphates, des sébaçates,
. des dérivés oxyéthylénés tels que les huiles oxyéthylénées, notamment les huiles végétales telles que l'huile de ricin; les huiles de silicone,
. des polymères hydrosolubles ou en dispersion aqueuse, ayant une température de transition vitreuse faible, inférieure à 25°C, de préférence inférieure à 15°C.

La quantité d'agent plastifiant est choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

La composition peut en outre comprendre des colorants et/ou des pigments utilisés de manière usuelle dans le domaine de la cosmétique et du maquillage.
Les pigments peuvent être présents dans la composition à raison de 0-20% en poids de la composition finale, et de préférence à raison de 1-5%. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

On peut également ajouter dans la composition selon l'invention tout additif connu tel que des agents épaississants, par exemple des argiles, des gommes, des silices, les dérivés cellulosiques, un polymère synthétique tel qu'un polymère acrylique ou un polymère associatif de type polyuréthanne; une gomme naturelle telle que la gomme xanthane; des agents d'étalement; des dispersants; des conservateurs; des agents antimousses; des agents mouillants; des filtres UV; des parfums; des charges; des actifs cosmétiques ou pharmaceutiques; des hydratants; des vitamines et leurs dérivés; des matières biologiques et leurs dérivés.
Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Le pH de la composition finale obtenue est de préférence inférieur à 9. Cette composition doit bien entendu être apte à se déposer sur un support tel que la peau ou les muqueuses.
La composition selon l'invention peut se présenter sous forme fluide, gélifiée, semi-solide, pâte souple, voire solide telle que de stick ou bâton.
Elle trouve en particulier une application en tant que produit de maquillage, notamment en tant que rouge à lèvres, fond de teint, fard à joues ou fard à paupières, ou encore eye-liner. On peut également envisager une application dans le domaine des compositions de soin, des compositions solaires, des compositions dermatologiques ou encore des compositions pharmaceutiques à appliquer sur la peau et/ou les muqueuses.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur de 300 microns avant séchage.
Après séchage pendant 24 heures, à 30°C et sous une humidité relative de 50%, on obtient un film ayant une épaisseur d'environ 100 microns; on mesure alors sa dureté à 30°C et 50% d'humidité relative.

### Exemple 1

On prépare des dispersions aqueuses comprenant des polymères ayant différentes duretés. On mesure la dureté du film obtenu et l'on apprécie la tenue du film sur les lèvres.
On obtient les résultats suivants :

| Polymère | Dureté | Appréciation visuelle |
|---|---|---|
| Polyuréthanne 1 | 170 | craquèle un peu; |
| SANCURE 815 | | se décolle après quelque temps |
| Polyuréthanne 2 | 165 | craquèle très vite au milieu des lèvres; |
| SANCURE 2060 | | se décolle sur les côtés |
| Polyuréthanne 3 | 115 | craquèle un peu; |
| NEOREZ R-974 | | se décolle après quelque temps |
| Polyuréthanne 4 | 104 | long à craqueler; |
| NEOREZ R-981 | | ne se décolle pas |
| Polyuréthanne 5 | 45 | très long à craqueler; |
| SANCURE 2255 | | ne se décolle pas |
| Polyuréthanne 6 | 33,5 | très long à craqueler; |
| SANCURE 878 | | ne se décolle pas |
| Polyuréthanne 7 | 24 | ne se craquèle pas; |
| 7 SANCURE 861 | | très souple |

### Exemple 2

On prépare un eye-liner ayant la composition suivante :

| | |
|---|---|
| . dispersion aqueuse de polyuréthanne (SANCURE 861) | 95 g |
| . pigment | 2 g |
| . agent plastifiant (glycérine) | 1,25 g |

On obtient une composition facile à appliquer sur le contour de l'oeil, qui donne un trait satiné et qui ne transfert pas et ne coule pas.

### Exemple 3

On prépare un rouge à lèvres ayant la composition suivante :

| | |
|---|---|
| . dispersion aqueuse de polyuréthanne (NEOREZ R-981) | 95 g |
| . pigment | 1 g |
| . agent plastifiant (glycérine) | 1,25 g |

On obtient une composition facile à appliquer sur les lèvres; le film obtenu est brillant; il ne transfert pas et ne migre pas dans les ridules; il résiste bien et suit le mouvement des lèvres.

### Exemple 4 (contre-exemple)

On prépare un rouge à lèvres ayant la composition suivante :

| | |
|---|---|
| . dispersion aqueuse de polyuréthanne (SANCURE 2060) | 95 g |
| . pigment | 1 g |
| . agent plastifiant (glycérine) | 1,25 g |

On obtient un film qui craquèle très rapidement après son application sur les lèvres.

## Revendications

1. Utilisation cosmétique dans une composition cosmétique susceptible d'être appliquée sur la peau, les semi-muqueuses et/ou les muqueuses, d'un système polymérique comprenant une dispersion aqueuse de particules de polymère filmogène, pour obtenir sur un support, un film de ladite composition ayant une dureté inférieure à 110, mesurée selon la norme ASTMD-43-66 ou NF-T-30-016.

2. Utilisation selon la revendication 1, dudit système polymérique pour obtenir un film de très bonne tenue et/ou qui ne transfère pas et/ou qui ne migre pas au cours du temps et/ou qui ne tache pas.

3. Utilisation selon l'une des revendications précédentes, dudit système polymérique pour obtenir un film souple et/ou élastique et/ou flexible sur la peau.

4. Utilisation selon l'une des revendications précédentes, dudit système polymérique pour obtenir un film qui suit les mouvements de la peau et/ou ne se craquèle pas et/ou ne se décolle pas.

5. Utilisation selon l'une des revendications précédentes, dudit système polymérique pour obtenir un film brillant.

6. Utilisation selon l'une des revendications précédentes, d'un système polymérique ou d'une composition le comprenant, pour maquiller la peau, les semi-muqueuses et/ou les muqueuses, en particulier les lèvres du visage, ledit système comprenant une dispersion aqueuse de particules de polymère filmogène tel qu'il conduit à l'obtention d'un film sur un support, ayant une dureté inférieure à 110.

7. Utilisation cosmétique selon l'une des revendications précédentes, d'un système polymérique ou d'une composition le comprenant, pour protéger et/ou traiter non thérapeutiquement la peau, les semi-muqueuses et/ou les muqueuses, en particulier les lèvres du visage, ledit système comprenant une dispersion aqueuse de particules de polymère filmogène et étant tel qu'il permette l'obtention d'un film ayant une dureté inférieure à 110, sur un support.

8. Utilisation pour la fabrication d'une composition destinée à traiter thérapeutiquement la peau, les semi-muqueuses et/ou les muqueuses, en particulier les lèvres du visage, d'un système polymérique ou d'une composition le comprenant, ledit système comprenant une dispersion aqueuse de particules de polymère filmogène et pour l'obtention sur un support d'un film ayant une dureté inférieure à 110, mesurée selon la norme ASTMD-4366 ou NF-T-30-016.

9. Utilisation selon l'une des revendications précédentes, dans un produit de maquillage tel qu'un rouge à lèvres, un fond de teint, un fard à joues ou fard à paupières, un eye-liner et/ou dans une composition de soin ou une composition solaire.

10. Utilisation selon la revendication 8, dans un produit de maquillage dans un produit de maquillage tel qu'un rouge à lèvres, un fond de teint, un fard à joues ou fard à paupières, un eye-liner et/ou dans une composition de soin, une composition dermatologique, une composition solaire, et/ou une composition pharmaceutique.

11. Utilisation selon l'une des revendications précédentes, dudit système polymérique pour obtenir sur un support un film ayant une dureté inférieure à 70, de préférence inférieure à 55, mesurée selon la norme ATSMD-43-66 ou NF-T-30-016.

12. Utilisation selon l'une des revendications précédentes, dans laquelle les particules de polymères en dispersion aqueuse ont une taille comprise entre 10-500 nm, de préférence comprise entre 20 et 150 nm.

13. Utilisation selon l'une des revendications précédentes, dans laquelle ledit système polymérique est tel qu'il conduit à l'obtention d'un film ayant une élongation supérieure à 200%, de préférence supérieure à 300%.

14. Utilisation selon l'une des revendications précédentes, dans laquelle la composition contient de 1 à 60 % en poids, de préférence 5 à 40 % en poids, de matière sèche de polymère filmogène.

15. Composition susceptible d'être appliquée sur la peau, les semi-muqueuses et/ou les muqueuses, comprenant un système polymérique qui comprend une dispersion aqueuse de particules de polymère filmogène, ledit système polymérique étant tel qu'il conduit à l'obtention sur un support d'un film ayant une dureté inférieure à 110, mesurée selon la norme ASTMD-43-66 ou NF-T-30-016.

16. Composition selon la revendication 15, se présentant sous la forme d'une composition de soin, d'une composition dermatologique, d'une composition solaire, d'une composition pharmaceutique et/ou d'un produit de maquillage tel qu'un rouge à lèvres, un fond de teint, un fard à joues ou fard à paupières, ou un eye-liner.

17. Composition selon l'une des revendications 15 à 16, ladite composition étant telle qu'elle permette, après application, l'obtention d'un film de très bonne tenue et/ou qui ne transfère pas et/ou qui ne migre pas au cours du temps et/ou qui ne tache pas.

18. Composition selon l'une des revendications 15 à 17, ladite composition étant telle qu'elle permette, après application, l'obtention d'un film brillant.

19. Composition selon l'une des revendications 15 à 18, caractérisée en ce qu'elle se présente sous forme de maquillage, notamment de rouge à lèvres ou de fond de teint, sans transfert.

20. Composition selon l'une des revendications 15 à 19, dans laquelle le système polymérique est tel qu'il permette l'obtention d'un film ayant une dureté inférieure à 70, de préférence inférieure à 55, mesurée selon la norme ATSMD-A3-66 ou NF-T-30-016.

21. Composition selon l'une des revendications 15 à 20, dans laquelle le système polymérique est tel qu'il permette l'obtention d'un film ayant une élongation supérieure à 200%, de préférence supérieure à 300%.

22. Composition selon l'une des revendications 15 à 21, dans laquelle le polymère filmogène est choisi parmi les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyesters, les polyesters amides, les polyesters à chaîne grasse, les polyamides, les résines époxyesters, les polymères ou copolymères acryliques et/ou vinyliques, les copolymères acryliques/silicones, les copolymères nitrocellulose/acryliques, les polymères d'origine naturelle éventuellement modifiés, les polymères hybrides et leurs mélanges.

23. Composition selon l'une des revendications 15 à 22, dans laquelle les particules de polymères en dispersion aqueuse ont une taille comprise entre 10-500 nm, de préférence comprise entre 20 et 150 nm.

24. Composition selon l'une des revendications 15 à 23, dans laquelle le système polymérique comprend en outre un agent plastifiant.

25. Composition selon la revendication 24, dans laquelle l'agent plastifiant est choisi parmi les glycols et leurs dérivés; les esters de glycérol; les dérivés de propylène glycol; des esters d'acides tels que des citrates, des phtalates, des adipates, des carbonates, des tartrates, des phosphates, des sébaçates; des dérivés oxyéthylénés tels que des huiles; des polymères hydrosolubles ou en dispersion aqueuse, ayant une température de transition vitreuse faible, inférieure à 25°C, de préférence inférieure à 15°C; et leurs mélanges.

26. Composition selon l'une des revendications 15 à 25, comprenant en outre des colorants et/ou des pigments.

27. Composition selon la revendication 26, dans laquelle les pigments sont présents à raison de 0-20% en poids, et de préférence 1-5% en poids.

28. Composition selon l'une des revendications 26 à 27, dans laquelle les pigments sont choisis parmi les dioxydes de titane, de zirconium ou de cérium, les oxydes de Zinc, de fer ou de chrome, le bleu ferrique, le noir de carbone, les laques de baryum, strontium, calcium, aluminium.

## Claims

1. Cosmetic use, in a cosmetic composition capable of being applied to the skin, the semimucosae and/or the mucosae, of a polymeric system which includes an aqueous dispersion of particles of film-forming polymer, in order to obtain, on a support, a film of the said composition which has a hardness lower than 110, measured according to the standard ASTMD-43-66 or NF-T-30-016.

2. Use according to Claim 1 of the said polymeric system, in order to obtain a film of very good staying power and/or which does not transfer and/or which does not migrate in the course of time and/or which does not stain.

3. Use according to either of the preceding claims of the said polymeric system, in order to obtain a film which is supple and/or elastic and/or flexible on the skin.

4. Use according to one of the preceding claims of the said polymeric system, in order to obtain a film which follows the movements of the skin and/or does not crack and/or does not lift off.

5. Use according to one of the preceding claims of the said polymeric system, in order to obtain a glossy film.

6. Use according to one of the preceding claims of a polymeric system or of a composition including it, for making up the skin, the semimucosae and/or the mucosae, in particular the lips, the said system including an aqueous dispersion of particles of film-forming polymer such that it leads to the production of a film, on a support, which has a hardness lower than 110.

7. Cosmetic use according to one of the preceding claims of a polymeric system or of a composition including it, for protecting and/or treating nontherapeutically the skin, the semimucosae and/or the mucosae, in particular the lips, the said system including an aqueous dispersion of particles of film-forming polymer and being such that it makes it possible to obtain a film which has a hardness lower than 110, on a support.

8. Use, for the manufacture of a composition intended to treat therapeutically the skin, the semimucosae and/or the mucosae, in particular the lips, of a polymeric system or of a composition including it, the said system including an aqueous dispersion of particles of film-forming polymer and in order to obtain, on a support, a film which has a hardness lower than 110, measured according to the standard ASTMD-4366 or NF-T-30-016.

9. Use according to one of the preceding claims, in a make-up product such as a lipstick, a foundation, a blusher, an eyeshadow or an eyeliner and/or in a care composition or an antisun composition.

10. Use according to Claim 8, in a make-up product such as a lipstick, a foundation, a blusher, an eyeshadow or an eyeliner and/or in a care composition, a dermatological composition, an antisun composition and/or a pharmaceutical composition.

11. Use according to one of the preceding claims of the said polymeric system, in order to obtain, on a support, a film which has a hardness lower than 70, preferably lower than 55, measured according to the standard ASTMD-43-66 or NF-T-30-016.

12. Use according to one of the preceding claims, in which the particles of polymers in aqueous dispersion have a size of between 10 and 500 nm, preferably between 20 and 150 nm.

13. Use according to one of the preceding claims, in which the said polymeric system is such that it leads to the production of a film which has an elongation greater than 200 %, preferably greater than 300 %.

14. Use according to one of the preceding claims, in which the composition contains from 1 to 60% by weight, preferably 5 to 40% by weight, of film-forming polymer solids.

15. Composition capable of being applied to the skin, the semimucosae and/or the mucosae, including a polymeric system which includes an aqueous dispersion of particles of film-forming polymer, the said polymeric system being such that it leads to the production, on a support, of a film which has a hardness lower than 110, measured according to the standard ASTMD-43-66 or NF-T-30-016.

16. Composition according to Claim 15, which is in the form of a care composition, a dermatological composition, an antisun composition, a pharmaceutical composition and/or a make-up product such as a lipstick, a foundation, a blusher, an eyeshadow or an eyeliner.

17. Composition according to one of Claims 15 to 16, the said composition being such that it makes it possible after application to obtain a film of very good staying power and/or which does not transfer and/or which does not migrate in the course of time and/or which does not stain.

18. Composition according to one of Claims 15 to 17, the said composition being such that it makes it possible after application to obtain a glossy film.

19. Composition according to one of Claims 15 to 18, characterized in that it is in the form of make-up, in particular lipstick or foundation, which does not transfer.

20. Composition according to one of Claims 15 to 19, in which the polymeric system is such that it makes it possible to obtain a film which has a hardness lower than 70, preferably lower than 55, measured according to the standard ASTMD-43-66 or NF-T-30-016.

21. Composition according to one of Claims 15 to 20, in which the polymeric system is such that it makes it possible to obtain a film which has an elongation greater than 200 %, preferably greater than 300 %.

22. Composition according to one of Claims 15 to 21, in which the film-forming polymer is chosen from anionic, cationic, nonionic or amphoteric polyurethanes, acrylic polyurethanes, polyvinylpyrrolidone polyurethanes, polyester polyurethanes, polyether polyurethanes, polyureas, polyesters, polyesteramides, polyesters containing a fatty chain, polyamides, epoxy ester resins, acrylic and/or vinyl polymers or copolymers, acrylic/silicone copolymers, nitrocellulose/acrylic copolymers, optionally modified polymers of natural origin, hybrid polymers and mixtures thereof.

23. Composition according to one of Claims 15 to 22, in which the particles of polymers in aqueous dispersion have a size of between 10 and 500 nm, preferably between 20 and 150 nm.

24. Composition according to one of Claims 15 to 23, in which the polymeric system additionally includes a plasticizing agent.

25. Composition according to Claim 24, in which the plasticizing agent is chosen from glycols and their derivatives, glycerol esters, propylene glycol derivatives, esters of acids, such as citrates, phthalates, adipates, carbonates, tartrates, phosphates and sebacates, oxyethylenated derivatives such as oils, polymers which are water-soluble or in aqueous dispersion, which have a low glass transition temperature, lower than 25°C, preferably lower than 15°C, and mixtures thereof.

26. Composition according to one of Claims 15 to 25, additionally including dyes and/or pigments.

27. Composition according to Claim 26, in which the pigments are present in a proportion of 0-20 % by weight and preferably 1-5 % by weight.

28. Composition according to one of Claims 26 to 27, in which the pigments are chosen from titanium, zirconium or cerium dioxides, zinc, iron or chromium oxides, ferric blue, carbon black and barium, strontium, calcium and aluminium lakes.

## Patentansprüche

1. Kosmetische Verwendung eines Polymersystems, das eine wäßrige Dispersion von Partikeln eines filmbildenden Polymers enthält, in einer kosmetischen Zusammensetzung, die auf die Haut, die Semi-Schleimhäute und/oder die Schleimhäute aufgetragen werden kann, um auf einem Träger einen Film dieser Zusammensetzung mit einer Härte unter 110 herzustellen, wobei die Härte gemäß der Norm ASTMD-43-66 oder NF-T-30-016 bestimmt wird.

2. Verwendung eines Polymersystems nach Anspruch 1, um einen Film herzustellen, der sehr gut haftet und/oder sich nicht überträgt und/oder im Laufe der Zeit keine Migration zeigt und/oder keine Flecken verursacht.

3. Verwendung eines Polymersystems nach einem der vorhergehenden Ansprüche, um einen Film herzustellen, der weich und/oder elastisch und/oder flexibel auf der Haut ist.

4. Verwendung eines Polymersystems nach einem der vorhergehenden Ansprüche, um einen Film herzustellen, der den Bewegungen der Haut folgt und/oder nicht rissig wird und/oder sich nicht ablöst.

5. Verwendung eines Polymersystems nach einem der vorhergehenden Ansprüche, um einen glänzenden Film herzustellen.

6. Verwendung eines Polymersystems nach einem der vorhergehenden Ansprüche oder einer Zusammensetzung, die dieses System enthält, um die Haut, die Semi-Schleimhäute und/oder die Schleimhäute und insbesondere die Lippen zu schminken, wobei das System eine wäßrige Dispersion von Partikeln eines filmbildenden Polymers enthält, so daß auf einem Träger ein Film mit einer Härte unter 110 hergestellt wird.

7. Kosmetische Verwendung eines Polymersystems nach einem der vorhergehenden Ansprüche oder einer Zusammensetzung, die dieses System enthält, um die Haut, die Semi-Schleimhäute und/oder die Schleimhäute und insbesondere die Lippen zu schützen und/oder nicht therapeutisch zu behandeln, wobei das System eine wäßrige Dispersion von Partikeln eines filmbildenden Polymers enthält, so daß auf einem Träger ein Film mit einer Härte unter 110 hergestellt werden kann.

8. Verwendung eines Polymersystems oder einer Zusammensetzung, die dieses System enthält, zur Herstellung einer Zusammensetzung, die dazu bestimmt ist, die Haut, die Semi-Schleimhäute und/oder die Schleimhäute und insbesondere die Lippen therapeutisch zu behandeln, wobei das System eine wäßrige Dispersion von Partikeln eines filmbildenden Polymers enthält, und zur Herstellung eines Films auf einem Träger, der eine Härte unter 110 aufweist, wobei die Härte gemäß der Norm ASTMD-43-66 oder NF-T-30-016 bestimmt wird.

9. Verwendung nach einem der vorhergehenden Ansprüche in einem Produkt zum Schminken, wie einem Lippenstift, Make-up, Wangenrouge, Lidschatten oder Eyeliner, und/oder in einer Zusammensetzung zur Pflege oder einer Zusammensetzung zum Sonnenschutz.

10. Verwendung nach Anspruch 8 in einem Produkt zum Schminken, wie einem Lippenstift, Make-up, Wangenrouge, Lidschatten oder Eyeliner, und/oder in einer Zusammensetzung zur Pflege, einer dermatologischen Zusammensetzung, einer Zusammensetzung zum Sonnenschutz und/oder einer pharmazeutischen Zusammensetzung.

11. Verwendung eines Polymersystems nach einem der vorhergehenden Ansprüche, um auf einem Träger einen Film herzustellen, der eine Härte unter 70 und vorzugsweise unter 55 aufweist, wobei die Härte gemäß der Norm ASTMD-43-66 oder NF-T-30-016 bestimmt wird.

12. Verwendung nach einem der vorhergehenden Ansprüche, wobei die Polymerpartikel in wäßriger Dispersion eine Größe im Bereich von 10 bis 500 nm und vorzugsweise 20 bis 150 nm aufweisen.

13. Verwendung nach einem der vorhergehenden Ansprüche, wobei das Polymersystem zur Herstellung eines Films führt, der eine Dehnung über 200 % und vorzugsweise über 300 % aufweist.

14. Verwendung nach einem der vorhergehenden Ansprüche, wobei die Zusammensetzung 1 bis 60 Gew.-% und vorzugsweise 5 bis 40 Gew.-% Trockensubstanz des filmbildenden Polymers enthält.

15. Zusammensetzung, die auf die Haut, die Semi-Schleimhäute und/oder die Schleimhäute aufgetragen werden kann und die ein Polymersystem enthält, das eine wäßrige Dispersion von Partikeln eines filmbildenden Polymers enthält, wobei das Polymersytem zur Herstellung eines Films auf einem Träger führt, der eine Härte unter 110 aufweist, wobei die Härte gemäß der Norm ASTMD-43-66 oder NF-T-30-016 bestimmt wird.

16. Zusammensetzung nach Anspruch 15, die in Form einer Zusammensetzung zur Pflege, einer dermatologischen Zusammensetzung, einer Zusammensetzung zum Sonnenschutz, einer pharmazeutischen Zusammensetzung und/oder eines Produkts zum Schminken, wie beispielsweise als Lippenstift, Make-up, Wangenrouge, Lidschatten oder Eyeliner, vorliegt.

17. Zusammensetzung nach einem der Ansprüche 15 und 16, wobei durch die Zusammensetzung nach dem Auftragen ein Film erhalten wird, der sehr gut haftet und/oder sich nicht überträgt und/oder im Laufe der Zeit keine Migration zeigt und/oder keine Flecken verursacht.

18. Zusammensetzung nach einem der Ansprüche 15 bis 17, wobei durch die Zusammensetzung nach dem Auftragen ein glänzender Film hergestellt werden kann.

19. Zusammensetzung nach einem der Ansprüche 15 bis 18, dadurch gekennzeichnet, daß sie in Form eines Schminkprodukts vorliegt, insbesondere als Lippenstift oder Make-up ohne Transfer.

20. Zusammensetzung nach einem der Ansprüche 15 bis 19, wobei durch das Polymersystem ein Film hergestellt werden kann, der eine Härte unter 70 und vorzugsweise unter 55 aufweist, wobei die Härte gemäß der Norm ASTMD-43-66 oder NF-T-30-016 bestimmt wird.

21. Zusammensetzung nach einem der Ansprüche 15 bis 20, wobei durch das Polymersystem ein Film hergestellt werden kann, der eine Dehnung über 200 % und vorzugsweise über 300 % aufweist.

22. Zusammensetzung nach einem der Ansprüche 15 bis 21, wobei das filmbildende Polymer unter den anionischen, kationischen, nichtionischen oder amphoteren Polyurethanen, Polyurethan-Acrylsäure-Polymeren, Polyurethan-Polyvinyl-pyrrolidonen, Polyester-Polyurethanen, Polyether-Polyurethanen, Polyharnstoffen, Polyestern, Polyesteramiden, Polyestern mit Fettkette, Polyamiden, Epoxyesterharzen, Acryl- und/oder Vinylpolymeren, Acryl- und/oder Vinylcopolymeren, Acryl/Siliconcopolymeren, Nitrocellulose/Acrylcopolymeren, Polymeren natürlichen Ursprungs, die gegebenenfalls modifiziert sind, Hybridpolymeren und deren Gemischen ausgewählt ist.

23. Zusammensetzung nach einem der Ansprüche 15 bis 22, wobei die Polymerpartikel in wäßriger Dispersion eine Größe im Bereich von 10 bis 500 nm und vorzugsweise im Bereich von 20 bis 150 nm aufweisen.

24. Zusammensetzung nach einem der Ansprüche 15 bis 23, wobei das Polymersystem ferner einen Weichmacher enthält.

25. Zusammensetzung nach Anspruch 24, wobei der Weichmacher unter den Glykolen und ihren Derivaten; Glycerinestern; Propylenglykolderivaten; Estern von Säuren, wie Citraten, Phthalaten, Adipaten, Carbonaten, Tartraten, Phosphaten, Sebacaten; Ethylenoxidderivaten, wie Ölen; wasserlöslichen Polymeren oder Polymeren in wäßriger Dispersion mit einer niedrigen Glasübergangstemperatur unter 25 °C und vorzugsweise unter 15 °C und deren Gemischen ausgewählt ist.

26. Zusammensetzung nach einem der Ansprüche 15 bis 25, die ferner Färbemittel und/oder Pigmente enthält.

27. Zusammensetzung nach Anspruch 26, wobei die Pigment in Mengenanteilen von 0 bis 20 Gew.-% und vorzugsweise 1 bis 5 Gew.-% vorliegen.

28. Zusammensetzung nach einem der Ansprüche 15 bis 27, wobei die Pigmente unter Titandioxid, Zirconiumdioxid, Cerdioxid, den Oxiden von Zink, Eisen und Chrom Eisenblau, Ruß und den Lacken von Barium, Strontium, Calcium und Aluminium ausgewählt sind.
